**Europäisches Patentamt**

**European Patent Office**

**Office européen des brevets**

(11) Veröffentlichungsnummer: **0 033 458**
**B1**

(12) **EUROPÄISCHE PATENTSCHRIFT**

(45) Veröffentlichungstag der Patentschrift:
**27.04.83**

(21) Anmeldenummer: **81100326.8**

(22) Anmeldetag: **17.01.81**

(51) Int. Cl.³: **C 08 G 18/32,** C 07 C 127/15,
C 07 C 127/19, C 07 C 127/24,
C 08 G 18/80

(54) **Verfahren zur Herstellung von modifizierten Polyisocyanaten und ihre Verwendung als Aufbaukomponente bei der Herstellung von Polyurethankunststoffen.**

(30) Priorität: **31.01.80 DE 3003543**

(43) Veröffentlichungstag der Anmeldung:
**12.08.81 Patentblatt 81/32**

(45) Bekanntmachung des Hinweises auf die Patenterteilung:
**27.04.83 Patentblatt 83/17**

(84) Benannte Vertragsstaaten:
**BE DE FR GB IT**

(56) Entgegenhaltungen:
**EP-A-0 003 505**
**DE-A-2 032 547**
**DE-A-2 261 065**
**DE-A-2 609 995**
**DE-A-2 804 375**

(73) Patentinhaber: **BAYER AG, Zentralbereich Patente, Marken und Lizenzen, D-5090 Leverkusen 1, Bayerwerk (DE)**

(72) Erfinder: **von Bonin, Wulf, Dr., Mendelsohnstrasse 30, D-5090 Leverkusen (DE)**
Erfinder: **Kleimann, Helmut, Dr., Mettlacher Strasse 6, D-5090 Leverkusen (DE)**
Erfinder: **Freitag, Hans-Albrecht, Dr., Mülheimer Strasse 135, D-5070 Bergisch-Gladbach (DE)**

Verfahren zur Herstellung von modifizierten Polyisocyanaten und ihre Verwendung als Aufbaukomponente bei der Herstellung von Polyurethankunststoffen

Die Erfindung betrifft ein neuartiges Verfahren zur Herstellung von modifizierten Polyisocyanaten durch Umsetzung von organischen Polyisocyanaten mit bestimmten, nachstehend näher beschriebenen organischen Polyaminen, sowie die Verwendung der Verfahrensprodukte als Aufbaukomponente bei der Herstellung von Polyurethankunststoffen nach dem Isocyanat-Polyadditionsverfahren.

Die Umsetzung von Polyisocyanaten mit primären oder sekundären Aminen zu Harnstoffen, bzw. Polyharnstoffen, ist als solche bekannt. Diese Reaktion verläuft im Vergleich zur Umsetzung von Polyisocyanaten mit primären oder sekundären Alkoholen wesentlich stürmischer, insbesondere, wenn ohne Verdünnungsmittel gearbeitet wird und ist thermisch oftmals nur schwer kontrollierbar (vgl. beispielsweise die diesbezüglichen Ausführungen in GB-A-1 263 609). So muss auch bei der Herstellung von Harnstoff-modifizierten Polyisocyanaten gemäss DE-A-2 032 547, d.h. durch Umsetzung von organischen Polyisocyanaten mit sekundären Polyaminen entweder unter Mitverwendung von Verdünnungsmitteln oder, wie aus den Ausführungsbeispielen ersichtlich, durch allmähliches Zutropfen des Polyamins zum Ausgangspolyisocyanat dafür Sorge getragen werden, dass die Reaktion nicht zu stürmisch verläuft.

Grundlage des nachstehend näher beschriebenen erfindungsgemässen Verfahrens ist die überraschende Beobachtung, dass dann, wenn man technisches Polyisocyanat, z.B. ein Toluylendiisocyanat-Isomerengemisch, mit technischem Ethylendiamin oder Diethylentriamin verrührt, die bekannte und zu erwartende heftige und quasi unkontrollierbare Reaktion einsetzt, dass aber dann, wenn man z.B. Triethylentetramin oder Tetraethylenpentamin als Aminkomponente verwendet, nur eine sehr schwache, gut kontrollierbare thermische Reaktion festzustellen ist; und zwar auch dann, wenn die eingesetzte Menge an Aminstickstoff vergleichbar ist.

Mit Hilfe der thermisch problemlos, d.h. ohne Mitverwendung von Verdünnungsmitteln bzw. ohne Einsatz von technisch aufwendigen Vorrichtungen zur Beherrschung der Reaktionswärme durchführbaren erfindungsgemässen Umsetzung von Polyisocyanaten mit den nachstehend näher beschriebenen speziellen Polyaminen lassen sich technische und reine, insbesondere bei Raumtemperatur flüssige Polyisocyanate und deren Gemische leicht in situ zu Suspensionen von Polyharnstoffen in eben diesen Polyisocyanaten modifizieren. Hierbei erreicht man vorzugsweise eine oftmals gewünschte veränderte (d.h. erhöhte) Viskosität und Rheologie der Isocyanate, bzw. Polyisocyanate, wodurch in vielen Fällen bei der Verarbeitung dieser Polyisocyanate zu geschäumten oder ungeschäumten, elastischen oder harten Kunststoffen Vorteile gesehen werden. Ausserdem führt die Verwendung der nachstehend näher beschriebenen erfindungsgemässen Verfahrensprodukte als Aufbaukomponente bei der Herstellung von Polyurethankunststoffen zu oft vorteilhaft modifizierten Endprodukten, so dass durch die erfindungsgemässe Bereitstellung der neuen Verfahrensprodukte die Variationsmöglichkeiten der technischen Polyurethanchemie erheblich erweitert werden.

Gegenstand der vorliegenden Erfindung ist ein Verfahren zur Herstellung von modifizierten Polyisocyanaten, durch Umsetzung bei 5 bis 150°C von a) organischen Polyisocyanaten mit b) Polyaminen, wobei die Menge der Komponente b) 0,1 bis 50 Gew.%, bezogen auf Gesamtgemisch, beträgt, mit der Massgabe, dass das Äquivalentverhältnis zwischen Isocyanatgruppen der Komponente a) und Aminogruppen der Komponente b) stets grösser als 2:1 ist, dadurch gekennzeichnet, dass man als Polyaminkomponente b) Polyalkylenpolyamine, ausgewählt aus der Gruppe bestehend aus Triethylentetramin, Tetraethylenpentamin, Tripropylentetramin und Tetrapropylenpentamin, sowie den technischen Gemischen dieser Polyamine, verwendet.

Gegenstand der vorliegenden Erfindung ist auch die Verwendung der nach diesem Verfahren erhältlichen modifizierten Polyisocyanate als Aufbaukomponente bei der Herstellung von Polyurethankunststoffen nach dem Isocyanat-Polyadditionsverfahren.

Für das erfindungsgemässe Verfahren sind als Ausgangsmaterialien a) im Prinzip alle beliebigen organischen Polyisocyanate geeignet, wie sie beispielsweise in US-A-4 058 492, Spalte 3, Zeile 33 bis Spalte 4, Zeile 61 bzw. in den dort zitierten Literaturstellen beschrieben sind. Zu den bevorzugten Ausgangskomponenten a) gehören Diisocyanate der Formel

$$R(NCO)_2$$

in welcher
R für einen zweiwertigen, gesättigten aliphatischen Kohlenwasserstoffrest mit 6 bis 10 Kohlenstoffatomen, einen zweiwertigen, gesättigten cycloaliphatischen Kohlenwasserstoffrest mit 4 bis 15 Kohlenstoffatomen, einem zweiwertigen aromatischen Kohlenwasserstoffrest mit 6 bis 17 Kohlenstoffatomen oder einen Xylylenrest steht, wobei zwischen den beiden Isocyanatgruppen jeweils mindestens 2 Kohlenstoffatome angeordnet sind.

Typische Beispiele für derartige bevorzugte Diisocyanate sind Hexamethylendiisocyanat, Dodecamethylendiisocyanat, Cyclobutandiisocyanat, 1-Methyl-2,4-diisocyanato-cyclohexan, 1-Isocyanato-3,3,5-trimethyl-5-methyl-5-isocyanatomethyl-cyclohexan (IPDI), 4,4'-Diisocyanato-dicyclohexylmethan, 2,4-Diisocyanatotoluol, dessen technischen Gemische mit 2,6-Diisocyanatotoluol, 4,4'-Diisocyanato-diphenylmethan, dessen technische Gemische mit 2,2'- und 2,4'-Diisocyanatodiphenylmethan und p-Xylylendiisocyanat. Die entsprechenden flüssigen Diisocyanate sind bevorzugt. Gemische der beispielhaft genannten Polyisocyanate können auch als Ausgangskomponente a) eingesetzt werden. 2,4-Diisocyanatotoluol bzw. dessen technischen Gemische mit bis zu 35 Gew.-%, bezogen auf Gesamtgemisch, an 2,6-Diisocyanatotoluol sind die erfindungsgemäss besonders bevorzugten Ausgangsverbindungen a). Von besonderem Interesse sind ebenfalls Abmischungen solcher difunktioneller Isocyanate mit bis zu 50 Gew.-%, bezogen auf Gesamt-Polyisocyanatgemisch, an höherfunktionellen Polyisocyanaten, insbesondere solcher wie sie durch Phosgenierung von Anilin/Formaldehyd-Kondensaten in an sich bekannter Weise zugänglich sind.

Bei der erfindungsgemäss einzusetzenden Ausgangskomponente b) handelt es sich um Polyalkylenpolyamine bzw. technischen Polyalkylenpolyamin-Gemische. Zu den erstgenannten Polyalkylenpolyaminen gehören beispielsweise Triethylentetramin, Tetraethylenpentamin, Tripropylentetramin oder Tetrapropylenpentamin. Zu den letztgenannten Polyalkylenpolyamin-Gemischen gehören die technischen Gemische der zuletzt beispielhaft genannten Polyalkylenpolyamine, die neben den genannten Polyaminen auch untergeordnete Mengen an anderen Polyaminen wie z.B. N-(2-Aminoethyl)-piperazin, N,N'-Bis-(2-aminoethyl)-piperazin, N-(2-Aminopropyl)-piperazin oder N,N'-Bis-(2-aminopropyl)-piperazin und/oder kettenverzweigte Isomere der genannten Polyamine enthalten können. Technisches Triethylentetramin und technisches Tetraethylenpentamin sind die besonders bevorzugten Ausgangskomponenten b).

Das erfindungsgemässe Verfahren wird so durchgeführt, dass man zwischen 5 und 150°C, vorzugsweise aber bei ca. 15 bis 75°C, insbesondere bei Raumtemperatur, die Polyisocyanatkomponente a) vorlegt und dann unter gutem Durchmischen die Aminkomponente b) hinzufügt. Da nur eine geringe Wärmetönung beobachtet wird, kann sehr schnell gearbeitet werden, was als technisch besonders vorteilhaft gewertet werden kann. Die Aminkomponente b) wird bezogen auf die Gesamtmischung in Mengen von 0,1 bis 50 Gew.-%, vorzugsweise 0,5 bis 10 Gew.-%, eingesetzt, wobei jedoch stets darauf geachtet werden muss, dass das Äquivalentverhältnis zwischen Isocyanatgruppen der Komponete a) und primären bzw. sekundären Aminogruppen der Komponente b) grösser als 2:1 ist. Nach kurzem intensivem Durchmischen der Ausgangskomponenten erhält man eine Suspension des Isocyanat- und Harnstoffgruppen aufweisenden Umsetzungsproduktes in überschüssiger Ausgangskomponente a).

Die erfindungsgemässe Umsetzung kann auch unter Mitverwendung von anderen, gegenüber Isocyanatgruppen reaktionsfähige Wasserstoffatome aufweisenden Reaktionspartnern für die Komponente a) durchgeführt werden. Beispiele hierfür sind insbesondere gegebenenfalls Ethergruppen aufweisende aliphatische Polyole des Molekulargewichtsbereichs 62-7000 oder einfacher Polyamine wie Ethylendiamin oder Propylendiamin. Auch im Falle der Mitverwendung derartiger zusätzlicher Reaktionspartner für die Komponente a) muss darauf geachtet werden, dass das Äquivalentverhältnis, bezogen auf alle Isocyanatgruppen der Komponente a) und alle gegenüber Isocyanatgruppen reaktionsfähige Gruppen, grösser als 2:1 ist. Die Mitverwendung derartiger zusätzlicher Reaktionspartner für die Komponente a) ist weniger bevorzugt. Die Mitverwendung von geringen Mengen an einfachen Polyaminen der beispielhaft genannten Art ist jedoch selbstverständlich dann ohne weiteres möglich, wenn die obengenannten Bedingungen bezüglich des Gehalts der Komponente b) an basischen Stickstoffatomen im statistischen Mittel erfüllt bleibt. Andernfalls, d.h. bei Verwendung grösserer Mengen an einfachen Polyaminen der beispielhaft genannten Art ist mit den eingangs genannten nachteilhaften Begleiterscheinungen zu rechnen.

Die erfindungsgemässe Umsetzung kann auch in Gegenwart von anorganischen oder organischen Füllstoffen und/oder in Gegenwart von in der Polyurethanchemie üblichen, inerten Zusatzmitteln durchgeführt werden. Diese Hilfs- und Zusatzmittel können selbstverständlich den erfindungsgemässen Verfahrensprodukten auch nach ihrer Herstellung einverleibt werden.

Die erfindungsgemässen Verfahrensprodukte können im Anschluss an ihre Herstellung selbstverständlich auch den an sich bekannten Modifizierungsreaktionen für organische Polyisocyanate z.B. den an sich bekannten Carbodiimidisierung-, Allophanatisierungs- oder Präpolymerisierungs-Reaktionen zugeführt werden.

Die erfindungsgemässen Verfahrensprodukte stellen Suspensionen von aus den Komponenten a) und b) gebildeten, Isocyanat- und Harnstoffgruppen aufweisenden Umsetzungsprodukten in überschüssiger Polyisocyanatkomponente a) dar. Sie weisen zumeist gegenüber der reinen Ausgangskomponente a) eine erhöhte Viskosität und bisweilen auch thixotrope Eigenschaften auf.

Die erfindungsgemässen Verfahrensprodukte können gegebenenfalls mit üblichen Polyisocyanaten abgemischt werden. Sie stellen wertvolle Ausgangsmaterialien zur Herstellung von Polyurethankunststoffen nach dem an sich bekannten Isocyanat-Polyadditionsverfahren dar. Sie werden hierzu als Polyisocyanatkomponente in Kombination mit den in der Polyurethanchemie üblichen Reaktionspartnern in den üblichen Mengenverhältnissen (mit)verwendet.

Die nachfolgenden Beispiele dienen zur weiteren Erläuterung der Erfindung. Alle Mengenangaben in «Teilen» beziehen sich auf Gewichtsteile.

Vergleichsbeispiel A

1425 Teile eines technischen Gemischs aus 80 Gew.-% 2,4- und 20 Gew.-% 2,6-Toluylendiisocyanat werden bei Raumtemperatur in eine 2 l Polyethylenflasche gegeben und mit einem Schnellrührer gerührt. Dann giesst man 75 Teile Ethylendiamin hinzu. Sofort beginnt eine heftige Reaktion die zu einem Temperaturanstieg auf 96°C führt.

Vergleichsbeispiel B

Es wird wie bei Vergleichsbeispiel A gearbeitet, als Amin nimmt man jetzt jedoch technisches Diethylentriamin. Es setzt sofort eine heftige Reaktion ein und die Temperatur steigt auf über 70°C an. Gleichzeitig ist eine Verquallung und Klumpenbildung zu beobachten.

In beiden Fällen zeigen die Vergleichsbeispiele die bekannte und zu erwartende heftige Reaktion zwischen aliphatischen Aminen und aromatischen Isocyanaten.

Beispiel 1

1425 Teile des in Vergleichsbeispiel A verwendeten Polyisocyanats werden in der dem Vergleichsbeispiel analogen Weise mit 75 Teilen technischem Triethylentetramin umgesetzt. Dieses Amin enthält neben (gaschromatographisch bestimmt) 70,4 Gew.-% linearem Polyamin 14,4 Gew.-% verzweigte Isomere und 9,0 Gew.-% Cyclisierungsprodukte (insbesondere N,N'-Bis-(2-aminoethyl)-piperazin und 6,2 Gew.-% weitere Nebenprodukte. Dies entspricht einem NCO/NH-Äquivalentverhältnis von ca. 9:1. Es ist keinerlei heftige Reaktion zu beobachten, vielmehr steigt nach ca. 5 Minuten die Temperatur langsam auf ca. 35°C und es bildet sich eine feinteilige leicht thixotrope Suspension des farblosen Reaktionsproduktes im Polyisocyanat. Nach ca. 10 Minuten wird das Rühren beendet. Nach 60 Minuten wird das inzwischen abgekühlte Reaktionsgemisch noch einmal kräftig durchgeschüttelt. Die entstandene Suspension hat eine bei 20°C gemessene Viskosität von ca. 8000 mPas.

Verwendet man die doppelte oder halbe Aminmenge, ist der Reaktionsverlauf nahezu gleichartig. Man erhält jeweils eine thixotrope Polyharnstoffsuspension im Polyisocyanat.

Beispiel 2

Es wird wie in Beispiel 1 unter Verwendung der gleichen Gewichtsmengen der Ausgangsmaterialien gearbeitet, wobei jedoch als Amin technisches Tetraethylenpentamin verwendet wird, das zu 45,9 Gew.-% aus linearem, zu 16,2 Gew.-% aus verzweigtem und zu 32,2 Gew.-% aus cyclischen Polyaminen besteht und eine Restfraktion von 5,7 Gew.-% weiterer Amine enthält. In diesem Falle steigt die Reaktionstemperatur auf ca. 25°C.

Die Beispiele 1 und 2 zeigen eine unerwartet milde Reaktion zwischen den reaktiven aromatischen Polyisocyanaten und Polyethylenpolyaminen mit mehr als 3 basischen Stickstoffatomen, die es ohne Schwierigkeiten gestattet, mit einfachsten Mitteln Suspensionen von Umsetzungsprodukten aus Polyalkylenpolyaminen mit mehr als 3 basischen Stickstoffatomen durch in situ-Reaktion in Polyisocyanaten herzustellen, d.h. diese Isocyanate zu modifizieren.

Beispiel 3

Es wird wie in Beispiel 1 unter Verwendung der gleichen Gewichtsmengen der Ausgangsmaterialien gearbeitet, mit dem Unterschied, dass hier technisches Isophorondiisocyanat (IPDI) anstelle des im Beispiel 1 verwendeten Toluylendiisocyanats verwendet wird. Man erhält unter schwacher Erwärmung eine feinteilige Suspension des Reaktionsproduktes aus Isophorondiisocyanat und Triethylentetramin mit einer Viskosität von ca. 4000 mPas/20°C.

Beispiel 4

100 Teile eines flüssigen Polyisocyanatgemisches der Diphenylmethanreihe, welches durch Phosgenierung von Anilin/Formaldehyd-Kondensaten in an sich bekannter Weise hergestellt worden ist, welches einen Gehalt an Isocyanatgruppen von 31 Gew.-% und eine Viskosität bei 20°C von 650 mPas aufweist, werden bei Raumtemperatur mit 20 Gew.-Teilen des technischen Triethylentetramins aus Beispiel 1 intensiv verrührt. Eine nennenswerte Temperaturerhöhung findet bei dieser Umsetzung nicht statt. Es wird vielmehr ein breiartiges Gemisch erhalten, welches sich im Laufe von einigen Stunden verfestigt.

Eine Wiederholung des Versuchs unter Verwendung von lediglich 10 Gew.-Teilen des technischen Triethylentetramins führt zu einem auf ca. 5000 mPas/20°C verdickten Polyisocyanatgemisch, welches sich ebenso wie die in den vorgehenden Beispielen beschriebenen Verfahrensprodukte für die Weiterverarbeitung zu Polyurethankunststoffen eignet.

Beispiel 5

9850 Teile eines technischen Gemisches aus 65 Gew.-% 2,4- und 35 Gew.-%, 2,6-Toluylendiisocyanat werden in einem Rührgefäss bei 35°C mit 150 Teilen des in Beispiel 1 verwendeten technischen Triethylentetramins versetzt, wobei 15 Minuten lang intensiv gerührt wird.

Nach 24 Stunden wird an der entstandenen Suspension bei 22°C eine Viskosität von 690 mPas gemessen (Isocyanat: 10 mPas).

Beispiel 6

4925 Teile des in Beispiel 5 verwendeten Isocyanats werden mit der gleichen Gewichtsmenge eines durch Phosgenierung eines Anilin/Formaldehyd-Kondensats gewonnenen Polyisocyanatgemischs der Diphenylmethanreihe der Viskosität 200 mPas./20°C abgemischt. Diese Mischung wird unter intensivem Rühren bei 40°C mit 150 Teilen Triethylentetramin versetzt. Man rührt ca. 15 Minuten und steigert die Temperatur auf 65°C mit einem Wasserbad. Dann wird abgekühlt. Nach 24 Stunden wird eine Viskosität von 1900 mPas bei 22°C gemessen, das Ausgangsgemisch hat eine Viskosität von ca. 25 mPas.

Beispiel 7

Analog Beispiel 1 werden 1500 Teile eines Gemischs aus 80 Gew.-% des in Vergleichsbeispiel A verwendeten Isocyanates und 20 Gew.-% des in Beispiel 6 verwendeten technischen Mehrkernisocyanatgemisches unter intensivem Rühren bei Raumtemperatur mit 30 Teilen Triethylentetramin umgesetzt. Das Ausgangsisocyanatgemisch hat eine Viskosität von ca. 10 mPas, die entstandene Suspension hat nach 40 Stunden eine Viskosität bei 20°C von 3200 mPas.

Die gemäss Beispielen 5 bis 7 erhältlichen Suspensionen haben einen leicht thixotropen Charakter und als Ausgangsmaterialien zur Herstellung von Polyurethankunststoffen, z.B. Polyurethanschaumstoffen, nach konventionellen Rezepturen geeignet.

## Patentansprüche

1. Verfahren zur Herstellung von modifizierten Polyisocyanaten, durch Umsetzung zwischen 5 und 150°C von a) organischen Polyisocyanaten mit b) Polyaminen, wobei die Menge der Komponente b) 0,1 bis 50 Gew.-%, bezogen auf Gesamtgemisch, beträgt, mit der Massgabe, dass das Äquivalentverhältnis zwischen Isocyanatgruppen der Komponente a) und Aminogruppen der Komponente b) stets grösser als 2:1 ist, dadurch gekennzeichnet, dass man als Polyaminkomponente b) Polyalkylenpolyamine, ausgewählt aus der Gruppe bestehend aus Triethylentetramin, Tetraethylenpentamin, Tripropylentetramin und Tetrapropylenpentamin, sowie den technischen Gemischen dieser Polyamine, verwendet.

2. Verfahren gemäss Anspruch 1, dadurch gekennzeichnet, dass man als Komponente a) Diisocyanate der Formel

$R(NCO)_2$

in welcher
R für einen zweiwertigen, gesättigten aliphatischen Kohlenwasserstoffrest mit 6 bis 10 Kohlenstoffatomen, einen zweiwertigen, gesättigten cyclophatischen Kohlenwasserstofrest mit 4 bis 15 Kohlenstoffatomen, einen zweiwertigen aromatischen Kohlenwasserstoffrest mit 6 bis 17 Kohlenstoffatomen oder einen Xylylenrest steht, wobei zwischen den beiden Isocyanatgruppen jeweils mindestens 2 Kohlenstoffatome angeordnet sind, verwendet.

3. Verfahren gemäss Ansprüchen 1 bis 2, dadurch gekennzeichnet, dass man als Komponente a) 2,4-Diisocyanatotoluol oder dessen technischen Gemische mit 2,6-Diisocyanatotoluol und als Komponente b) Triethylentetramin verwendet.

4. Verwendung der gemäss Ansprüchen 1 bis 3 erhältlichen modifizierten Polyisocyanate als Aufbaukomponente bei der Herstellung von Polyurethankunststoffen nach dem Isocyanat-Polyadditionsverfahren.

## Claims

1. Process for the preparation of modified polyisocyanates by reacting, at between 5 and 150°C, a) organic polyisocyanates with b) polyamines, the quantity of component b) bein 0.1 to 50% by weight, based on the total mixture, with the proviso that the equivalent ratio between isocyanate groups of the component a) and amino groups of the component b) is always greater than 2:1, characterised in that as polyamine component b) polyalkylene polyamines, selected from the group consisting of triethylene tetramine, tetraethylene pentamine, tripropylene tetramine and tetrapropylene pentamine and the commercial mixtures of these polyamines, are used.

2. Process according to Claim 1, characterised in that diisocyanates of the formula

$R(NCO)_2$

are used as component a)
in which
R represents a difunctional, saturated aliphatic hydrocarbon radical having 6 to 10 carbon atoms, a difunctional, saturated cycloaliphatic hydrocarbon radical having 4 to 15 carbon atoms, a difunctional aromatic hydrocarbon radical having 6 to 17 carbon atoms or a xylylene radical, at least 2 carbon atoms being in each case positioned between the two isocyanate groups.

3. Process according to Claims 1 to 2, characterised in that 2,4-diisocyanatotoluene or commercial mixtures thereof with 2,6-diisocyanatotoluene is used as component a) and triethylene tetramine is used as component b).

4. Use of the modified polyisocyanates obtainable according to Claims 1 to 3 as a starting component in the preparation of polyurethane plastics according to the isocyanate polyaddition process.

## Revendications

1. Procédé pour produire des polyisocyanates modifiés, par réaction entre 5 et 150°C, de (a) des polyisocyanates organiques avec (b) des polyamines, la quantité du composant (b) représentant 0,1 à 50% en poids, par rapport au mélange total, étant bien entendu que le rapport entre les équivalents des groupes isocyanates du composant (a) et des groupes amino du composants (b) est toujours supérieur à 2:1, procédé caractérisé en ce qu'on utilise comme composant polyamine (b) des polyalkylènepolyamines, choisies dans l'ensemble constitué par la triéthylènetétramine, la tétraéthylènepentamine, la tripropylènetétramine et la tétrapropylènepentamine, ainsi que les mélanges techniques de ces polyamines.

2. Procédé selon la revendication 1, caractérisé en ce qu'on utilise comme composant (a) des diisocyanates de formule:

$R(NCO)_2$

dans laquelle
R représente un reste d'hydrocarbures aliphatiques saturés divalents comportant 6 à 10 atomes de carbone, un reste d'hydrocarbures cycloali- phatiques saturés divalents comportant 4 à 15 atomes de carbone, un reste d'hydrocarbures aromatiques divalents comportant 6 à 17 atomes de carbone ou un reste xylylène, 2 atomes de carbone au moins étant à chaque fois interposés entre les deux groupes isocyanates.

3. Procédé selon l'une des revendications 1 et 2, caractérisé en ce qu'on utilise comme composant (a) du 2,4-diisocyanatotoluène ou ses mélanges techniques avec du 2,6-diisocyanatetoluè-ne, et, comme composant (b), de la triéthylènété-tramine.

4. Utilisation des polyisocyanates modifiés, que l'on peut obtenir selon l'une des revendications 1 à 3, comme composants pour la production des matières plastiques du type polyurétha-ne selon le procédé de poly-addition d'isocya-nates.